# EUROPEAN PATENT APPLICATION

(11) **EP 0 533 321 A2**
(43) Date of publication of application: **24.03.1993**
(21) Application number: 92306485.1
(22) Date of filing: 15.07.1992
(51) Int. Cl.: A61B 17/22

(54) **Expanding atherectomy device**

(30) Priority: 22.07.1991 US 734375
(71) Applicant: DOW CORNING WRIGHT CORPORATION, Arlington Tennessee (US)
(72) Inventor: Fine, Michael Jay, Coral Springs, Florida (US); Kortenbach, Jürgen Andrew, Fort Lauderdale, Florida (US)
(74) Representative: Laredo, Jack Joseph

(57) **Abstract**

According to the invention, there is provided an intravascular catheter (10) having a rotating working head (20) comprising an elongated flexible jacket having opposed proximal and distal ends and a central passageway (12) extending between the ends. The working head is located at the distal end of the jacket. A concentrically wound drive cable (13) extends through the passageway of the jacket, having a driving end operatively connected to the working head for rotating it and a driven end operatively connected to a source of high-speed motion. The working head is rotatably mounted in a bearing (25) and further attached to the drive cable. The working head further includes a rotating tip with a plurality of axially-extending wires (23) attached to the driving end of the cable. The wires are actuated to reversibly expand from an initial retracted position with the wires radially compressed within the distal end of the jacket, to an activated position distally of the jacket, with the wires being radially expanded to a larger diameter than that of the jacket.

## Description

The invention generally relates to intravascular catheters having rotating working heads useful in removing occlusions from a vessel, particularly an artery or vein.

During an intravascular procedure, it is typically necessary to utilize some type of mechanical device to remove the occlusion from a vessel. Generally, a recanalization catheter including a rotatable working head with a fixed diameter is used for engaging and cutting the blockage from the vessel wall. As the catheter is advanced, the vessel increases or decreases in diameter necessitating the use of several different tip sizes to complete the procedure. Fixed diameter catheters of this type are available from Dow Corning Wright-Theratek of Miami Lakes Florida, under the name "TRAC-WRIGHT", in various size designations.

To change the working diameter during the procedure, the catheter is removed from the lumen of the vessel and a catheter having a larger or smaller working diameter corresponding to the changed lumen diameter is reinserted into the vessel.

U.S. Patent No. 4,895,560 to Papantonakos describes a milling tool for angioplasty comprising an actuator cylinder for increasing or decreasing the diameter of the tool. The tool further comprises a multiplicity of flexible, leaf-shaped flutes axially connected to a driving rod. The flutes are linearly displaceable with respect to the actuator cylinder, causing a change in the radial dimension of the flutes. As the rod is retracted by the actuator through a base plate the tool is compressed, forcing the flutes to bow outward thereby increasing the effective working diameter of the tool a corresponding amount. One disadvantage of this tool is that sharpened edges of the rotating flutes can damage vascular tissue. Moreover, the dimensional change occurs in response to static manual adjustments rather than operational conditions of use.

U.S. Patent No. 4,273,128 to Lary discloses cutting and dilating instrument for removing stenosis from a coronary artery. The instrument comprises a cannula with a flexible probe at the distal end thereof for guiding the instrument through the artery. A number of knife blades, extending radially outwardly from and welded or soldered to the cannula at its distal end, are inserted into the stenosis and manually rotated by the physician to remove the obstruction. The instrument can be fed through a catheter jacket and advanced forward from the jacket to expose the blades to the stenosis. However, one disadvantages of this catheter is that such blades can damage by cutting or abrading vascular tissue.

Accordingly, there remains a need for an intravascular catheter having a rotating head which is self-expandable to different effective working diameters while being relatively non-abrasive to vascular tissue.

According to the invention, there is provided an intravascular catheter comprising an elongated flexible jacket having opposed proximal and distal ends and a central passageway extending between the ends. A rotatable working head is located at the distal end of the jacket. A drive cable extends within the passageway and has a driving end rotatably coupled to the working head and a driven end operatively connected to a source of high-speed motion. The working head further includes a rotating tip with a plurality of axially-extending wires attached to the driving end of the cable. An actuator activates the wire pair to reversibly expand from an initial retracted position, with the wires radially compressed within the distal end of the jacket, to an advanced position distally of the jacket wherein the wires are radially expanded to a larger effective diameter than that of the jacket.

In a preferred embodiment, bearing means are provided for rotatably mounting the working head to the drive cable and retaining the wires within the jacket.

An advantage of the invention is safe, effective removal of occlusions by a tool without sharpened edges that could potentially damage vascular tissue.

Another advantage of the invention is the capability of adjusting the working head diameter without removing the catheter from the lumen of the vessel being recanalized, allowing the working head to effectively expand to a larger diameter than that of the lumen.

A further advantage of the invention is the ability to navigate the interventional device through the vascular system over a guide wire to the desired site therein, including infusion into and aspiration of fluids from the lumen through the jacket of the catheter.

A further advantage of the invention, in its preferred form, is a portable, self-contained and ergonomic hand-held controller which also includes the power source imparting rotational movement to the working head.

The invention may be better appreciated by reference to the attached drawings, which illustrate one or more preferred embodiments, wherein:
**FIG. 1** is an enlarged longitudinal sectional view of the distal end of a catheter, showing a working head with a plurality of wires in an extended position, according to the invention;
**FIG. 2** is a front view slowing the working head of the catheter of **FIG. 1**;
**FIG. 3** is a sectional view showing the catheter of **FIG. 1**, with the wires in a retracted position;

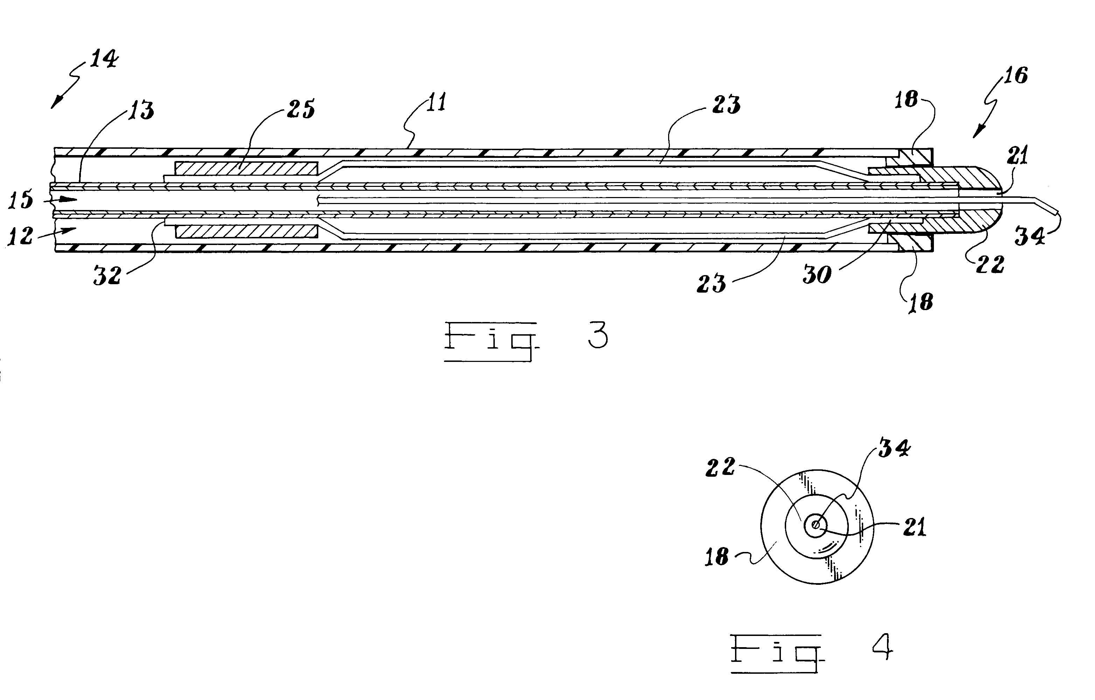

**FIG. 4** is a front view of the working head of **FIG 3**;

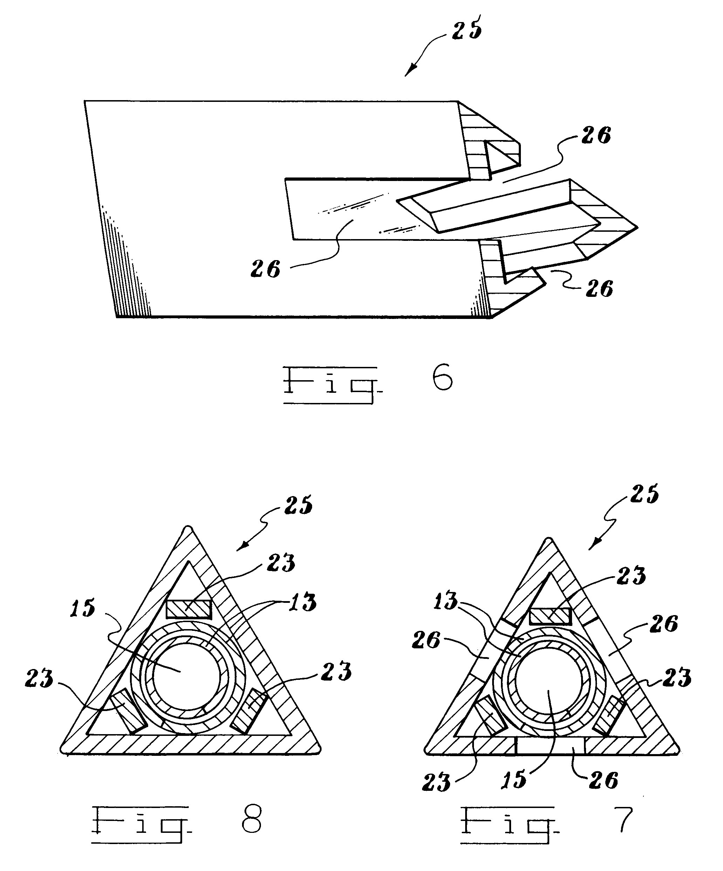

**FIG. 5** is a sectional view, taken along lines **2-2** of **FIG. 1** showing the preferred bearing means of the invention;
**FIG. 6** is a perspective view showing the bearing means of **FIG. 5**;
**FIG. 7** is a front view of the bearing means of **FIG. 5**;
**FIG. 8** is a rear view of the bearing means of **FIG 5**; and
**FIG. 9** is a sectional view, showing the preferred controller of the invention;
**FIG.** **10** is an enlarged sectional view from **FIG. 9**, showing the preferred connector sleeve of the invention.

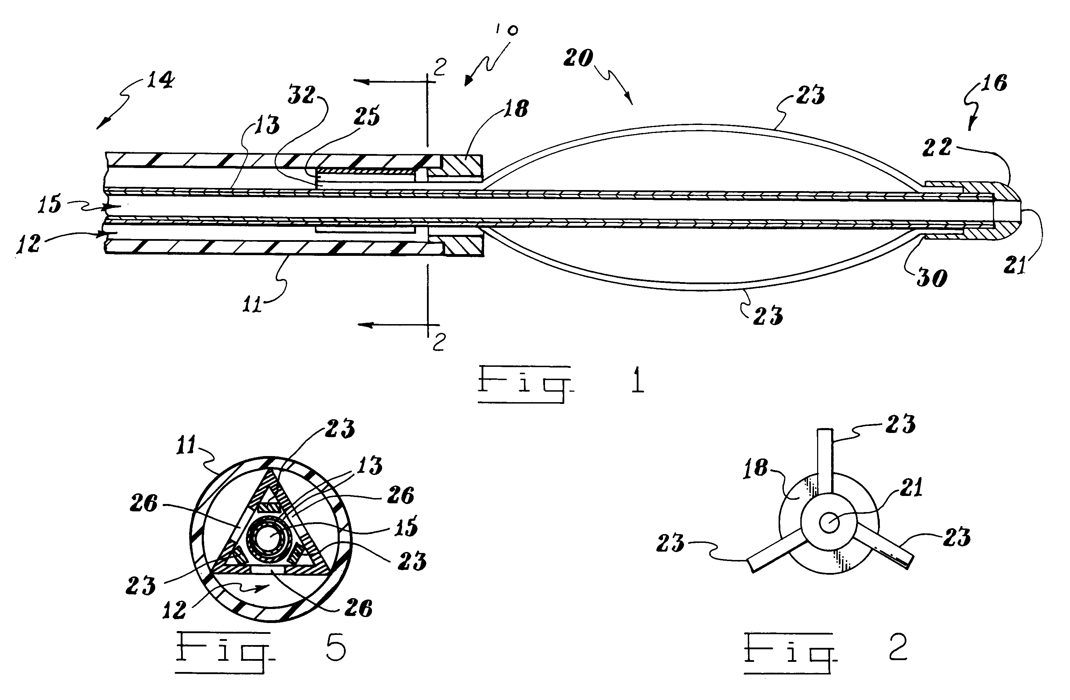

Referring to **FIGS. 1**, **2** and **3**, an intravascular catheter is generally shown at **10** for removing occlusions from the lumen of a vessel. As depicted in **FIG. 1**, the catheter **10** comprises a jacket **11** including a central passageway **12** within which a drive cable **13** extends between proximal **14** and distal **16** ends of the jacket **11**. A working head, generally indicated at **20**, is located at the distal end **16** of jacket **11** and a guideway **21** is formed in a tip **22** mounted to rotate as the working head is rotated by the drive cable **13**. The tip **22** further comprises a plurality of expandable wires **23** affixed to the drive cable **13**. The wires **23** have a distal end **30** rotatably connected to drive cable **13** and a proximal end **32** received within a bearing means **25**.

Referring again to **FIG. 1**, the working head **20** is shown in the fully-extended, i.e., "activated", position with the wires **23** expanded to a diameter larger than that of jacket **11**. The wires **23** are formed from flexible wire having a circular or polygonal cross-sectional area of about 0.026 mm². The rotating distal end **30** of the wires **23** are sandwiched between the tip **22** and the drive cable **13** and proximal end **32** sandwiched between the bearing means **25** and the drive cable **13** whereby both ends of the wires are fixedly welded in place. The tip **22** has a rounded front portion and the guideway **21** receives an axial guidewire **34** (**FIG. 3**) routed through a central core **15** of the drive cable **13** from the proximal end **14** of the jacket **11**.

Further referring to **FIG. 1**, the working head **20** is rotatably mounted within the bearing means **25**. A retainer band **18** glued to the distal **16** end of jacket **11** further affixes the bearing means **25** within the jacket. A cutaway section is removed from the bearing means **25** forming a plurality of ports **26** for suctioning stenosis debris through central passageway **12** for removal by an aspiration means (not shown).

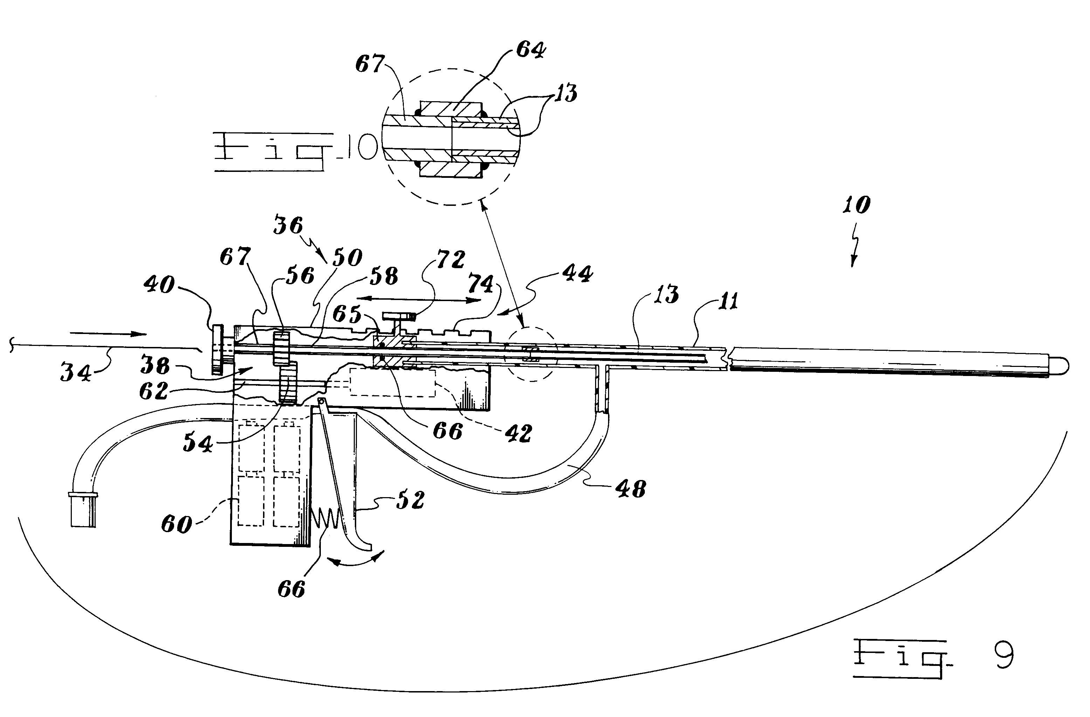

**FIGS. 6, 7** and **8** defines the generally triangular shape bearing means **25**, **FIG. 6** showing a perspective view of the ports **26**. **FIG. 7** shows a front view of the bearing means **25** indicating the wires **23** and drive cable **13** orientation. **FIG. 8** shows a rear view of the bearing means **25**, wires **23** and drive cable **13**.

As shown in **FIG. 1**, the working head **20** is rotated by drive cable **13**, which is preferably comprised of two concentrically wound metal flat ribbons that spiral about a common axis along the length of the cable. These metal ribbons are coupled to the output slaft of a source of high-speed motion and rotate in response to energization. The inner ribbon tends to expand outward and the outer ribbon tends to contract inward about the inner ribbon when energized, maintaining a central core **15** within the inner ribbon while transmitting torque to the working head **20**. The central core **15** provides a passage for guidewire **34**. An example of such a drive cable is found in U.S. Patent No. 4,917,085, assigned to the same owner as the present application.

Referring to **FIG. 9**, the catheter **10** is preferably coupled to a controller **36** comprising a offset gear drive assembly **38**, a guidewire hemostasis valve **40**, a DC motor **42**, an actuator mechanism **44**, an aspiration tube **48** and a trigger **52**. A housing **50**, made of polycarbonate or some other suitable material, encases the components of the actuator **36** and defines an ergonomic profile. The controller **36** is coupled to the drive cable **13** at the proximal end **14** of the jacket **11** to rotate the drive cable at high speeds, i.e., from 5,000 to 20,000 RPM. The offset gear drive assembly **38** further comprises a driving gear **54** coupled through shaft **62** to a DC motor **42**, engaging a driven gear **56** having an axial passageway **58** for accepting a hollow shaft **67**. The guidewire 34 is inserted through the hemostasis valve **40**, the housing **50** and hollow shaft **67**, thus allowing external manipulation of the guidewire.

The actuator mechanism **44** governs expansion/retraction of the wires **23** (**FIG. 9**), further comprising the hollow shaft **67**, a connector sleeve **64**, a slider knob **72**, a seal housing **65** and a slider guide **74** formed in the housing **50**. Again referring to **FIG. 9**, the hollow shaft **67** passes through the axial passageway **58** of driven gear **56** through seal housing **65**. An O-ring **66** surrounds the outside circumference of the hollow shaft **67** and forms a seal, between the shaft and the seal housing **65**, capable of maintaining a vacuum within the central passageway **12**, while allowing the shaft to slide within the seal housing. The adjoining ends of hollow slaft **67** and drive cable **13** are welded in place using the connector sleeve **64** (**FIG. 10**). The seal housing **65** is further connected to the slider knob **72**, extending through the slider guide **74**. The slider guide **74** is used to position and lock the wires **23** in an extended or retracted position, furthermore the slider guide is capable of stoppage in a multiple of positions between the fully-retracted and fully-extended.

In **FIG. 9**, the jacket **11** is shown attached to the housing **65**, thereby completely sealing the catheter. As the slider knob **72** is moved proximally, the jacket **11** slides axially over the drive cable **13** to expose the "activated" wires **23**. Alternatively, the device could operate to extend and retract the drive cable for activating the working head (not shown), as compared to the method described in this specification; however, sliding the jacket **11** relative to a fixed drive cable **13** simplifies the drive mechanism, using fewer parts and reducing the number of moving seals.

Batteries **60** power the DC motor **42**, actuated by depressing the trigger **52**, causing an electrical switch (not shown) to close. In the "off" position, the trigger **52** pinches close the aspiration tube **48** which removes debris from the work site. A spring **66** applies force to the trigger **52** pinching off the aspiration tube **48**. When the trigger **52** is in the activated position, aspiration tube **48** is opened and aspirated by a vacuum source (not shown).

In operation, the guidewire **34** is inserted percutaneously through an incision into the vessel, then progressively advanced to the location of the stenosis, preferably with the aid of visual monitoring by radiopaque dyes or an endoscope. The catheter **10**, having only the tip **22** exposed (retracted position), is then advanced along guidewire **34** so that the tip **22** is positioned near the location of the stenosis. The jacket **11** is retracted by the controller **36**, exposing the tip **22** and wires **23**, allowing the wires to expand to a radial diameter larger than that of the jacket (activated position). The wires **23** are advanced further into the stenosis and rotated, until contacting and removing the stenosis using a scraping non-abrasive action. The stenosis debris is then suctioned from the work situs through ports **26** and removed through central passageway **12** of the jacket **11** and finally aspirated through aspiration tube **48**.

## Claims

1. An intravascular catheter for removing occlusions from a vessel comprising:
(a) an elongated catheter jacket having opposed proximal and distal ends, defining a central passageway extending between the ends;
(b) a rotating working head located at the distal end of the jacket, having a drive cable extending through the passageway and operatively connected to a high-speed source of motion at the proximal end for rotating the cable at relatively high operating speeds;
(c) a bearing means for rotatably connecting the working head to the drive cable, the working head further includes a rotating tip with a plurality of axially-extending wires attached to the drive cable adjacent the distal end of the jacket, the wires are reversibly expandable from an initial retracted position with the wires radially compressed within the distal end of the jacket to an activated position outside the jacket, with the wires radially expanded to a larger diameter than that of the jacket;
(d) a controller for actuating the working head to adjust from an at least initial retracted position with a first effective working diameter to an extended position with a second diameter that is greater than the first.

2. The catheter of claim 1 wherein the drive cable further comprises two concentric.ally wound metal flat ribbons including a central passageway.

3. An intravascular catheter for removing occlusions from a vessel comprising:
(a) an elongated catheter jacket having opposed proximal and distal ends and a wall defining a central passageway extending between the ends;
(b) a rotating working head located at the distal end of the jacket, having a concentrically wound drive cable extending through the passageway and connected to a source of rotary power at the proximal end for rotating the cable at relatively high operating speeds;
(c) a bearing means for rotatably connecting the working head to the drive cable, the working head further includes a rotating tip with a plurality of axially-extending wires attached to the drive cable adjacent the distal end of the jacket, the wires are reversibly expandable from an initial retracted position with the wires radially compressed within the distal end of the jacket to an activated position outside the jacket, with the wires being radially expanded to a larger diameter than that of the jacket;
(d) a controller for actuating the working head to adjust from an at least initial retracted position with a first effective working diameter to an extended position with a second diameter that is greater than the first by retracting the catheter jacket.

4. The catheter of claim 3 further comprising an aspiration means located at the jacket distal end.

5. The catheter of claim 3 wherein the concentrically wound drive cable further comprises two close wound helical wrapped coils of wire, with one coil wrapped over the other in the opposite direction.

6. The catheter of claim 3 wherein the controller is a hand-holdable, self-contained unit further comprising aspiration means, a power means, a infusion means, a motor means and an actuator means.

7. The catheter of claim 6 further comprises an offset gear drive assemble for rotating the drive cable.

8. The catheter of claim 6 further comprises a hemostasis valve for sealing around the guidewire.

9. An intravascular catheter for removing occlusions from a vessel comprising, a tip having three wires with a common free end, a triangular-shaped bearing for rotatably supporting the fixed ends of the wires within the working head.
